# EUROPEAN PATENT SPECIFICATION

(11) **EP 2 893 956 B1**
(45) Date of publication and mention of the grant of the patent: **22.02.2017**
(21) Application number: 14150635.2
(22) Date of filing: 09.01.2014
(51) Int. Cl.: A61N 5/10

(54) **Radiation planning system**
Strahlungsplanungssystem
Système de planification de rayonnement

(43) Date of publication of application: 15.07.2015
(73) Proprietor: PTW - Freiburg Physikalisch-Technische Werkstätten Dr. Pychlau GmbH, 79115 Freiburg (DE)
(72) Inventor: Pychlau, Christian, 79100 Freiburg (DE)
(74) Representative: Maucher Jenkins Patent- und Rechtsanwälte

(56) References cited:
- DE-A1-102010 061 121

## Description

### Background

The invention relates to a method of determining a radiation distribution, a data processing system for implementing the method, and to a radiation planning system.

Radiation treatment of patients Is usually preceded by a radiation planning stage. Radiation planning involves the determination of radiation doses to which the patient is to be exposed during treatment. This may include a spatial distribution of the radiation dose and/or a distribution of the radiation dose over time. The spatial distribution and the distribution over time will generally be referred to as radiation distribution herein.

For the purpose of quality control, the radiation distribution determined in the radiation planning stage is compared with measurements of the actual radiation distribution during simulated treatment using a so-called phantom, i.e. a more or less body-equivalent device replacing a patient during a simulated radiotherapy and enabling measurements within the volume to be irradiated. Differences between the measurements and the predetermined radiation distribution can be taken into account when correcting the radiation treatment plan.

It is an object of the present invention to improve the radiation planning so that the differences between the predetermined radiation distribution and the actual (measured) radiation distribution are reduced to a minimum.

DE 102010061121 A1 describes a system pertaining to the features of the preamble of the independent claims. In particular, this document describes a radiation phantom device for validating radiation planning, which comprises at least one movement device for moving a first sub-region of the radiation phantom device relative to a second sub-region of the radiation phantom device.

### Summary of the invention

According to one aspect of the present Invention, there is provided a method of determining a radiation distribution, comprising: determining or estimating movements of a patient during an intended a radiation exposure; exposing a phantom to radiation, wherein the radiation exposure of the phantom corresponds to said intended radiation exposure of the patient; measuring first radiation values at respective first positions during the radiation exposure of the phantom; and calculating second radiation values at respective second positions based on said first radiation values and values of one or more parameters indicative of said movements of the patient.

The present invention is based on the realization that radiation planning can be improved by taking into account the movements of a patient during treatment. In particular, the present invention is directed to taking into account patient movements already in the radiation planning stage. Such movements may be movements of the entire or part of the patient, e.g. movements caused by breathing.

Patient movements are taken into account by simulating the movements of regions of interest of the phantom. For this purpose, the algorithm that is used to calculate the second radiation values from the first radiation values takes into account the movement of such regions of interest. Such algorithm can - in the easiest case - simply consist of the convolution of the four-dimensional set of dose measurement values with a corresponding four-dimensional set of motion vectors.

The first and second positions may be identical. This enables determining how a radiation dose at a given position changes from an initial value (the first radiation value) to a subsequent value (the second radiation value) due to patient movements.

Alternatively, the second positions may be different from the first positions. The second radiation values indicate whether a position of interest is exposed to sufficient radiation and/or whether surrounding tissue and vital organs at risk are subjected to an inacceptable dose level.

Also, the first and second positions may represent regions. The regions represented by the first and second positions may be identical, or they may entirely or partly overlap. In this embodiment, regions of interests, for example regions containing tissue to be destroyed, can be examined.

The first and second radiation values may represent radiation intensities or doses and may be included in a three-dimensional matrix. From such matrix, it is possible to extract and examine the radiation in selected planes and/or from selected angles.

In addition, it is possible to determine the distribution of the first and second radiation values over time. This enables the examination of selected periods or phases of the treatment.

In an embodiment of the invention, the first radiation values are measured through sensors placed at or in the phantom or a treatment couch on which the phantom is supported, wherein, during the radiation exposure of the phantom, radiation from a radiation source passes through at least a portion of the phantom before reaching or impinging on said sensors.

In an embodiment of the invention, the method comprises generating a depth dose or tissue phantom rate curve associated with the patient or a phantom representing the patient; adapting the depth dose or tissue phantom rate curve in accordance with the movements of a patient during a radiation exposure; and determining the second radiation values on the basis of the first radiation values and the adapted depth dose or tissue phantom rate curve. A depth dose or tissue phantom rate curve indicates the distribution of the radiation dose along the trajectory of the radiation. Accordingly, it is possible to obtain a relatively high number of radiation values distributed in three-dimensional space on the basis of a relatively low number of measurement values in a single plane. In particular, it is possible to derive values at positions along the trajectory above or underneath the plane in which the sensors are located. For example, the values in three-dimensional space can be obtained by multiplying the measurement values with associated values of the depth dose or tissue phantom rate curve.

Generally, patient movements may be measured, estimated or determined empirically. The movements may be represented by a mathematical function, e.g. a sinus or elliptical function indicative of periodical movements such as movements caused by breathing, wherein the amplitude is determined empirically. In another embodiment, the movements may be represented by values in a lookup table or matrix.

In an embodiment, the method comprises deriving the parameter values indicative of the patient movements from data obtained from a control device associated with an adjustable treatment couch, the control device to dynamically cause adjustments of the treatment couch in accordance with movements of a patient during treatment. By using data obtained from such control device it is possible to empirically determine patient movements without requiring additional equipment or measurements.

Alternatively, it is possible to derive the parameter values indicative of the patient movements from data obtained from a measurement device for measuring movements of the patient or parts of the patient during treatment, wherein the measurement device may comprise one or more acceleration sensors attached to the patient and/or an X-ray, magnetic resonance, laser and/or an ultrasound apparatus. This embodiment enables precisely determining patient movements in specific regions of interest.

According to another aspect of the present invention, there is provided a data processing system comprising: Means to obtain or store values of one or more parameters indicative of movements of a patient during a radiation exposure; means to obtain measurements of first radiation values at respective positions during a radiation exposure of a phantom; and means to determine second radiation values based on said first radiation values and said obtained or stored parameter values.

The data processing system may be included in a radiation therapy quality assurance (QA) system comprising an array of sensors located at different respective positions in or at a phantom and/or a treatment couch supporting the phantom, for measuring first radiation values during a radiation exposure of the phantom.

The radiation therapy QA system may further comprise one or more phantoms having at least some of said array of sensors located in or at the phantom. The phantom preferably has one or more predetermined characteristics that correspond to those of a patient who is to undergo radiation treatment. In particular, the phantom has essentially the same transmissibility or permeability characteristics for the type of radiation that is used for the radiation treatment of the patient.

Accordingly, the present invention may be advantageously implemented by or integrated with a quality control or management system consisting of or comprising a phantom for providing the first radiation values and software for calculating the second radiation values.

The present invention enables the recalculation of radiation doses over space and/or time during the planning stage of a radiation treatment, taking into account estimated movements of the patient during the treatment.

In an embodiment of the invention, a set of vectors indicative of the location of voxels of the patient is generated. This is done on the basis of a model, predetermined information and/or measurements. The vectors are 4-dimensional: three dimensions in space and one dimension in time. Initially, the value of all vectors is zero.

The set of vectors include information indicative of one or more of the following parameters:
- Deformation of the patient
- Possible movements of the patient
- Possible movements of the treatment couch
- Other relevant movements of deformations

Measurements of the stationary phantom indicate when and what the linear accelerator radiates, and in which direction. The measurements also provide 4-dimensional data: the dose is measured in one plane (2D), while the dose along the depth is reconstructed (1D); this is done over time (1D). At this stage neither the treatment couch nor the phantom needs to be moved, thus simplifying the measurements.

By means of such 4D dose distribution, the doses in the patient can be calculated, using the patient's CT. The CT used in this step does not include or reflect any patient movements or deformations. Such movements or deformations are taken into account in the next step. Using the 4D vectors, the patient CT is deformed. Using the 4D dose distribution, the dose distribution in respect of the deformed CT is calculated for each point in time. Thereby, the doses that the patient is exposed to due to his movements and deformations are obtained. From such dose distribution, the doses at the patient target volume (PTV) or at organs at risk (OAR) can be derived.

### Brief description of the drawings

An embodiment of the invention will now be described by reference to the drawings. In the drawings,
Fig. 1 illustrates the steps of a method according to an embodiment of the present invention; and
Fig. 2 illustrates schematically a radiation therapy QA system according to an embodiment of the invention.

### Detailed description of the drawings

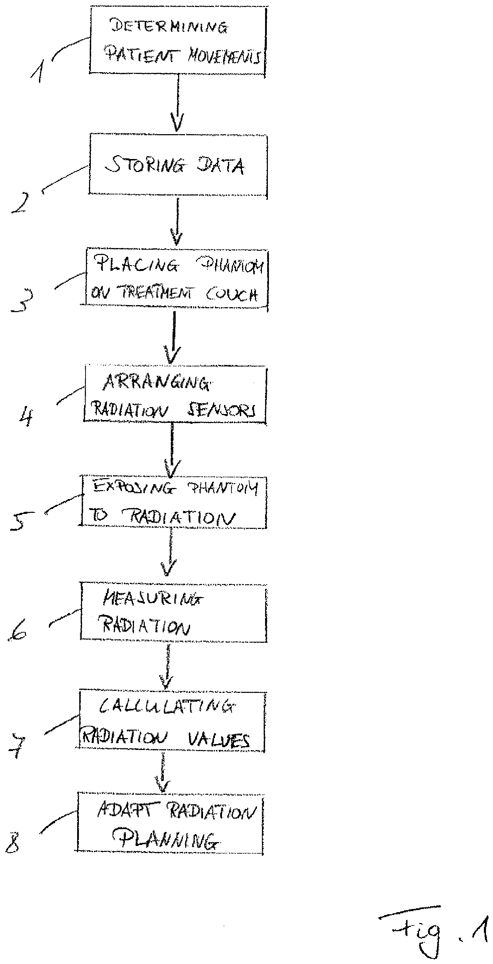

Fig. 1 illustrates the steps of a method according to an embodiment of the present invention. In step 1, the movements of a patient are measured. These can be the movements of a "sample" patient, or of the actual patient to be treated, on a treatment couch. The measurements can be performed by placing sensors on the patient, e.g. acceleration sensors, or by employing an ultrasound or X-ray apparatus.

In step 2, data indicative of the patient movements is stored. In particular, the data is stored in the memory of a data processing system that is provided to control and/or perform the steps of the described method.

In step 3, a phantom is placed on the treatment couch. The phantom should have the same or a similar basic anatomy as the patient to be treated. Also, the phantom should have essentially the same characteristics as the patient in terms of transmissibility or permeability for radiation.

In step 4, radiation sensors are placed in or at the phantom and/or the treatment couch so that radiation emitted by a radiation generator passes through portions of the phantom before reaching the sensors. In an embodiment, the sensors are integrated with the phantom.

In step 5, the phantom is exposed to radiation emitted by a radiation generator. The radiation doses, in particular the distribution of the radiation doses over time and/or space corresponds to the radiation doses intended for the radiation treatment of the patient.

In step 6, the radiation is measured by the sensors. The measurement values are stored in the memory of the data processing system.

In step 7, the measured patient movements and the measured radiation values are used to calculate second radiation values. In particular, the second radiation values are based on the measured radiation values and additionally reflect the movements of the patient.

In step 8, the second radiation values are used to adapt the radiation doses, in particular the distribution of the radiation doses over time and/or space, intended for the radiation treatment of the patient, thereby to eliminate effects caused by the patient movements.

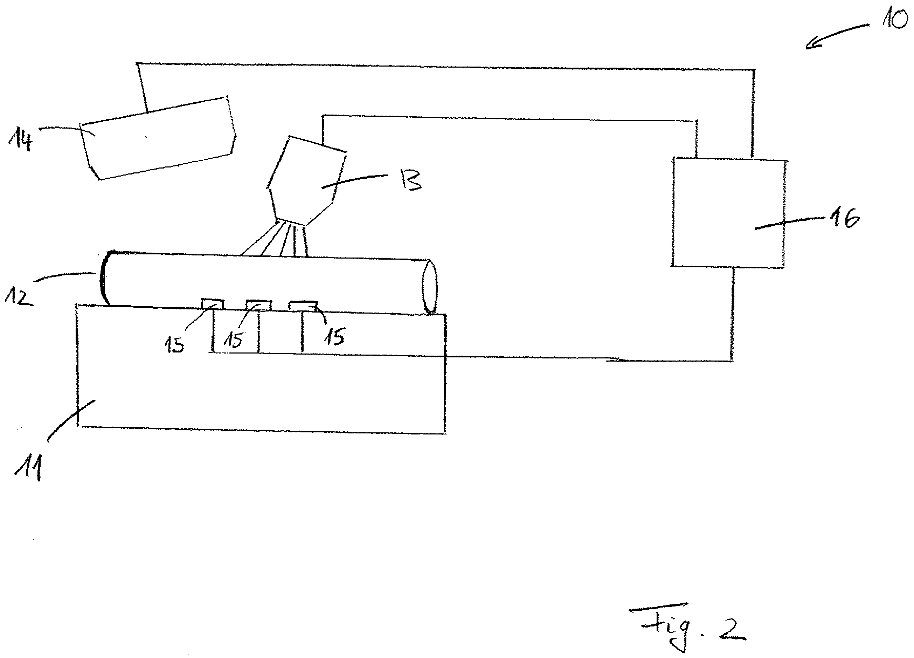

Fig. 2 illustrates schematically a radiation therapy QA system 10 according to an embodiment of the invention. The system 10 comprises a treatment couch 11 for supporting a phantom 12 or a patient. The system further comprising a radiation generator 13 and an apparatus 14, for example an X-ray, MR, laser or ultrasound apparatus, for measuring the movements of a patient on the treatment couch 11. In addition, the system 10 comprises a plurality of radiation sensors 15 at the treatment couch 11 or the phantom 12 for measuring the radiation emitted from the radiation generator 13 at predetermined positions. The radiation sensors 15 may be integral with the phantom 12.

The radiation generator 13, the apparatus 14 and the sensors 15 are connected to a data processing and control device 16. The data processing and control device 16 is arranged to cause some or all of the steps illustrated in Fig. 1 to be performed. In particular, when a patient is placed on the treatment couch 11, the data processing and control device 16 controls the apparatus 14 to detect and measure movements of the patient, e.g. movements caused by breathing, and to receive and store data indicative of the patient movements.

Subsequently, when the phantom 12 is placed on the treatment couch 11, the data processing and control device 16 controls the sensors 15 to measure the radiation, and receives and stores the measured radiation values.

The data processing and control device 16 uses the data indicative of the patient movements and the measured radiation values to calculate second radiation values by e.g. convoluting the measured radiation values with the corresponding displacement vectors previously determined.

This means that the four-dimensional set of dose data values, obtained e.g. by continuously measuring in one plane of a cylindrical phantom - which itself is always oriented perpendicular to the radiation beam's central axis by the control device - and continuously adding calculated (by depth dose curve or tissue phantom rate) values for the rest of the cylindrical phantom's volume, is displaced by an equally four-dimensional set of vectors determined from the patient movement data into a new four-dimensional set of dose data values distributed over the treatment time and the volume of the phantom. These can then be used for a more realistic appraisal of the treatment plan.

Accordingly, the second radiation values reflect expected movements by a patient. In other words, the second radiation values represent values that would have been measured if the phantom had moved or had been deformed in accordance with the measured patient movement.

The data processing and control device 16 compares the second radiation values with the measured radiation values. The difference between the measured radiation values and the second radiation values indicates the effect on the radiation at the sensors 15 caused by the patient movements. For example, if the second radiation value is higher than the measured radiation value, this indicates that more radiation than expected has passed through the corresponding portion of the phantom 12.

The data processing and control device 16 may be used to adapt a stored radiation distribution for use in a subsequent radiation treatment, for example to reduce the radiation directed to a given area if the second radiation values in that area indicate a higher-than-expected radiation.

## Claims

1. A method of determining a radiation distribution, comprising:
determining (1) or estimating movements of a patient during an intended radiation exposure;
exposing (5) a phantom to radiation, wherein the radiation exposure of the phantom corresponds to said intended radiation exposure of the patient; and
measuring (6) first radiation values at respective first positions during the radiation exposure of the phantom;
**characterized by**
calculating (7) second radiation values at respective second positions based on said first radiation values and values of one or more parameters Indicative of said movements of the patient.

2. The method of claim 1, wherein said first and second radiation values represent radiation intensities or doses.

3. The method of claim 1 or 2, wherein said first positions and said second positions are identical, respectively.

4. The method of any preceding claim, comprising arranging (4) sensors for measuring said first radiation values at or in the phantom or a treatment couch on which the phantom is supported, wherein, during said radiation exposure of the phantom, some of the radiation from a radiation source passes through at least a portion of the phantom before reaching said sensors.

5. The method of any preceding claim, comprising determining the radiation distribution over time.

6. The method of any preceding claim, comprising:
generating a depth dose or tissue phantom rate curve associated with the patient or a phantom representing the patient;
adapting the depth dose or tissue phantom rate curve in accordance with said movements; and
determining said second radiation values on the basis of said first radiation values and the adapted depth dose or tissue phantom rate curve.

7. The method of any preceding claim, comprising:
deriving the parameter values indicative of said movements from data obtained from a control device associated with an adjustable treatment couch, the control device to cause adjustments of the treatment couch in accordance with movements of a patient during treatment.

8. The method of any of claims 1 to 6 comprising:
deriving the parameter values indicative of said movements from data obtained from a measurement device for measuring movements of a patient during treatment, wherein the measurement device optionally comprises one or more acceleration sensors attached to the patient and/or an adjustable treatment couch supporting the patient and/or an X-ray, magnetic resonance, laser and/or an ultrasound apparatus.

9. The method of any preceding claim, wherein said parameter values indicative of the patient movements are based on data previously determined by another system.

10. A data processing system (16) comprising:
means to obtain or store values of one or more parameters indicative of movements of a patient during a radiation exposure; and
means to obtain measurements of first radiation values at respective positions during a radiation exposure of a phantom;
**characterized by**:
means to determine second radiation values based on said first radiation values and said obtained or stored parameter values.

11. A radiation treatment quality assurance system comprising:
an array of sensors (15) locatable at different respective positions In or at a phantom and/or a treatment couch supporting the phantom, for measuring first radiation values during a radiation exposure of the phantom; and
the data processing system (16) of claim 10.

12. The radiation treatment quality assurance system of claim 11, comprising at least one phantom (12) having at least some of said array of sensors (15) located in or at the phantom.

## Patentansprüche

1. Verfahren zum Bestimmen einer Strahlungsverteilung, umfassend:
Bestimmen (1) oder Abschätzen von Bewegungen eines Patienten während einer vorgesehenen Strahlenexposition;
Exponieren (5) eines Phantoms gegenüber Strahlung, wobei die Strahlenexposition des Phantoms der vorgesehenen Strahlenexposition des Patienten entspricht; und
Messen (6) erster Strahlungswerte an entsprechenden ersten Positionen während der Strahlenexposition des Phantoms;
**gekennzeichnet durch**
Berechnen (7) zweiter Strahlungswerte an entsprechenden zweiten Positionen auf der Grundlage der ersten Strahlungswerte und von Werten eines oder mehrerer Parameter, die für die Bewegungen des Patienten indikativ sind.

2. Verfahren gemäß Anspruch 1, wobei die ersten und zweiten Strahlungswerte Strahlungsintensitäten oder -dosen darstellen.

3. Verfahren gemäß Anspruch 1 oder 2, wobei die ersten Positionen und die zweiten Positionen identisch sind.

4. Verfahren gemäß einem der vorstehenden Ansprüche, umfassend Anordnen (4) von Sensoren zum Messen der ersten Strahlungswerte an oder in dem Phantom oder einer Behandlungsliege, von der das Phantom getragen wird, wobei während der Strahlenexposition des Phantoms etwas von der Strahlung aus einer Strahlenquelle durch wenigstens einen Teil des Phantoms tritt, bevor es die Sensoren erreicht.

5. Verfahren gemäß einem der vorstehenden Ansprüche, umfassend das Bestimmen der Strahlungsverteilung im Zeitverlauf.

6. Verfahren gemäß einem der vorstehenden Ansprüche, umfassend:
Erzeugen einer Tiefendosis- oder Gewebephantom-Ratenkurve, die mit dem Patienten oder einem Phantom, das den Patienten darstellt, verbunden ist;
Anpassen der Tiefendosis- oder Gewebephantom-Ratenkurve den Bewegungen entsprechend; und
Bestimmen der zweiten Strahlungswerte auf der Grundlage der ersten Strahlungswerte und der angepassten Tiefendosis- oder Gewebephantom-Ratenkurve.

7. Verfahren gemäß einem der vorstehenden Ansprüche, umfassend:
Ableiten der Parameterwerte, die für die Bewegungen indikativ sind, aus Daten, die von einer Steuervorrichtung erhaltenen sind, die mit einer einstellbaren Behandlungsliege verbunden ist, wobei die Steuervorrichtung Einstellungen der Behandlungsliege den Bewegungen eines Patienten während einer Behandlung entsprechend bewirkt.

8. Verfahren gemäß einem der Ansprüche 1 bis 6, umfassend:
Ableiten der Parameterwerte, die für die Bewegungen indikativ sind, aus Daten, die von einer Messvorrichtung zum Messen von Bewegungen eines Patienten während einer Behandlung erhalten sind, wobei die Messvorrichtung gegebenenfalls einen oder mehrere Beschleunigungssensoren, die an dem Patienten befestigt sind, und/oder eine einstellbare Behandlungsliege, die den Patienten trägt, und/oder eine Röntgen-, Magnetresonanz-, Laser- und/oder Ultraschallvorrichtung umfasst.

9. Verfahren gemäß einem der vorstehenden Ansprüche, wobei die Parameterwerte, die für die Patientenbewegungen indikativ sind, auf der Grundlage von Daten stehen, die zuvor von einem anderen System bestimmt wurden.

10. Datenverarbeitungssystem (16), umfassend:
Mittel zum Erhalten oder Speichern von Werten von einem oder mehreren Parametern, die für Bewegungen eines Patienten während einer Strahlenexposition indikativ sind; und
Mittel zum Erhalten von Messungen erster Strahlungswerte an entsprechenden Positionen während einer Strahlenexposition eines Phantoms;
**gekennzeichnet durch**:
Mittel zum Bestimmen zweiter Strahlungswerte auf der Grundlage der ersten Strahlungswerte und der erhaltenen oder gespeicherten Parameterwerte.

11. Strahlenbehandlungs-Qualitätssicherungssystem, umfassend:
ein Feld von Sensoren (15), die an verschiedenen entsprechenden Positionen in oder an einem Phantom und/oder einer Behandlungsliege, die das Phantom trägt, platzierbar sind, zum Messen erster Strahlungswerte während einer Strahlenexposition des Phantoms; und
das Datenverarbeitungssystem (16) gemäß Anspruch 10.

12. Strahlenbehandlungs-Qualitätssicherungssystem gemäß Anspruch 11, umfassend wenigstens ein Phantom (12), das wenigstens einige aus dem Feld von Sensoren (15) in oder an dem Phantom angeordnet aufweist.

## Revendications

1. Procédé pour déterminer une distribution de rayonnement, comprenant les opérations suivantes :
déterminer (1) ou estimer des mouvements d'un patient pendant une exposition envisagée à un rayonnement ;
exposer (5) un fantôme au rayonnement, l'exposition du fantôme au rayonnement correspondant à ladite exposition au rayonnement envisagée pour le patient ; et
mesurer (6) des premières valeurs de rayonnement à des premières positions respectives pendant l'exposition au rayonnement du fantôme ;
**caractérisé en ce que** :
l'on calcule (7) des deuxièmes valeurs de rayonnement à des deuxièmes positions respectives sur la base desdites premières valeurs de rayonnement et des valeurs d'un ou de plusieurs paramètre(s) indicatives desdits mouvements du patient.

2. Procédé selon la revendication 1, dans lequel lesdites premières et deuxièmes valeurs de rayonnement représentent des intensités ou des doses de rayonnement.

3. Procédé selon la revendication 1 ou 2, dans lequel lesdites premières positions et lesdites deuxièmes positions sont identiques, respectivement.

4. Procédé selon l'une quelconque des revendications précédentes, comprenant l'arrangement (4) de capteurs pour mesurer lesdites premières valeurs de rayonnement sur ou dans le fantôme ou une couche de traitement sur laquelle le fantôme est supporté, dans lequel, pendant ladite exposition au rayonnement du fantôme, une certaine quantité du rayonnement provenant d'une source de rayonnement passe à travers au moins une partie du fantôme avant d'atteindre lesdits capteurs.

5. Procédé selon l'une quelconque des revendications précédentes, comprenant la détermination de la distribution du rayonnement au cours du temps.

6. Procédé selon l'une quelconque des revendications précédentes, comprenant les étapes suivantes :
générer une courbe du taux de dose ou de tissu fantôme en profondeur associée au patient ou à un fantôme représentant le patient ;
adapter la courbe de taux de dose ou de tissu fantôme en profondeur conformément auxdits mouvements ; et
déterminer lesdites deuxièmes valeurs de rayonnement sur la base desdites premières valeurs de rayonnement et de la courbe de taux de dose ou de tissu fantôme en profondeur adaptée.

7. Procédé selon l'une quelconque des revendications précédentes, comprenant l'étape suivante :
dériver les valeurs de paramètres indicatives desdits mouvements à partir de données obtenues grâce à un dispositif de commande associé à une couche de traitement réglable, le dispositif de commande permettant de procéder à des ajustements de la couche de traitement conformément aux mouvements d'un patient pendant le traitement.

8. Procédé selon l'une quelconque des revendications 1 à 6, comprenant l'étape suivante :
dériver les valeurs de paramètres indicatives desdits mouvements à partir de données obtenues à partir d'un dispositif de mesure pour mesurer des mouvements d'un patient pendant le traitement, dans lequel le dispositif de mesure comprend en option un ou plusieurs détecteur(s) d'accélération attaché(s) au patient et/ou une couche de traitement réglable supportant le patient et/ou un appareil à rayons X, à résonance magnétique, à laser et/ou à ultrasons.

9. Procédé selon l'une quelconque des revendications précédentes, dans lequel lesdites valeurs de paramètres indicatives des mouvements du patient sont basées sur des données déterminées antérieurement par un autre système.

10. Système de traitement de données (16) comprenant :
des moyens pour obtenir ou stocker des valeurs d'un ou de plusieurs paramètre(s) indicatives de mouvements d'un patient pendant une exposition à un rayonnement ;
et
des moyens pour obtenir des mesures de premières valeurs de rayonnement à des positions respectives pendant une exposition au rayonnement d'un fantôme ;
**caractérisé par** :
des moyens pour déterminer des deuxièmes valeurs de rayonnement sur la base desdites premières valeurs de rayonnement et desdites valeurs de paramètres obtenues ou stockées.

11. Système d'assurance de qualité d'un traitement par rayonnement comprenant :
un réseau de capteurs (15) pouvant être localisé à différentes positions respectives dans ou sur un fantôme et/ou une couche de traitement supportant le fantôme, pour mesurer des premières valeurs de rayonnement pendant une exposition au rayonnement du fantôme ; et
le système de traitement de données (16) selon la revendication 10.

12. Système d'assurance de qualité d'un traitement par rayonnement selon la revendication 11, comprenant au moins un fantôme (12) muni d'au moins certains dudit réseau de capteurs (15) localisés dans ou sur le fantôme.
